# EUROPEAN PATENT SPECIFICATION

(11) **EP 3 962 863 B1**
(45) Date of publication and mention of the grant of the patent: **24.04.2024**
(21) Application number: 20729179.0
(22) Date of filing: 29.04.2020
(51) Int. Cl.: C01G 49/00, C01G 49/08, C01G 51/00, C09C 1/24, H01F 1/00, A61K 41/00

(54) **METHOD FOR THE GRAM-SCALE PREPARATION OF CUBIC FERRITE NANOCRYSTALS FOR BIOMEDICAL APPLICATIONS**
VERFAHREN ZUR HERSTELLUNG VON KUBISCHEN FERRIT-NANOKRISTALLEN GRAMM-MASSSTAB FÜR BIOMEDIZINISCHE ANWENDUNGEN
MÉTHODE DE PRÉPARATION À L'ÉCHELLE DU GRAMME DE NANOCRISTAUX CUBIQUES DE FERRITE POUR DES APPLICATIONS BIOMÉDICALES

(30) Priority: 30.04.2019 IT 201900006469
(43) Date of publication of application: 09.03.2022
(73) Proprietor: Fondazione Istituto Italiano di Tecnologia, 16163 Genova (IT)
(72) Inventor: PELLEGRINO, Teresa, 16145 Genova (IT); GAVILÁN RUBIO, Helena, 16143 Genova (IT); MAI, Binh Thanh, Nha Trang City (VN); CINGOLANI, Roberto, 16014 CeranesI (Genova) (IT)
(74) Representative: Comoglio, Elena
(86) International application number: PCT/IB2020/054028
(87) International publication number: WO 2020/222133

(56) References cited:
- US-A1- 2005 191 231
- US-A1- 2017 143 854
- PABLO GUARDIA ET AL: "One pot synthesis of monodisperse water soluble iron oxide nanocrystals with high values of the specific absorption rate", JOURNAL OF MATERIALS CHEMISTRY B, vol. 2, no. 28, 1 January 2014 (2014-01-01), page 4426, XP055657025, GB ISSN: 2050-750X, DOI: 10.1039/c4tb00061g cited in the application
- AN-HUI LU ET AL: "Magnetic Nanoparticles: Synthesis, Protection, Functionalization and Application", ANGEWANDTE CHEMIE, INTERNATIONAL EDITION, WILEY-VCH, DE, vol. 46, no. 8, 5 February 2007 (2007-02-05), pages 1222-1244, XP008128389, ISSN: 1433-7851, DOI: 10.1002/ANIE.200602866 [retrieved on 2007-02-05]

## Description

### Technical field

The present invention relates to a method for preparing nanoparticles having controlled structural and magnetic properties for biomedical applications, in particular for the application thereof as thermal mediators in magnetic hyperthermia.

### Prior art

Magnetic hyperthermia is a promising approach for the treatment of neoplastic diseases, which is able to bring about the selective killing of the tumor cells on account of the heat, without damaging the healthy cells at the same time. This treatment is based on the use of magnetic nanoparticles to bring about a localized increase in temperature in the target cancerous region by means of an increase in temperature of 42-45°C. The factors that determine the efficacy of this treatment are the alternating magnetic field (frequency and intensity) and the structural and colloidal properties of the nanoparticles used. The conditions of the alternating magnetic field are dictated by clinical conditions (in general, a frequency of 110 kHz and an intensity of between 10 and 24 kAm⁻¹ are used on patients). In particular, it is known that cubic magnetite (Fe₃O₄) and maghemite (γ-Fe₂O₃) nanocrystals show, owing to their anisotropic characteristics, exceptional behavior compared with the corresponding spherical nanoparticles during magnetic hyperthermia. The typical dose of spherical nanoparticles required for magnetic hyperthermia treatment of a patient is 7-12 mL of a solution containing 100-120 mg Fe/mL, while the cubic nanocrystals, which have specific absorption rate values (commonly called SAR) that are twenty times greater than those of the spherical nanoparticles, would make it possible to considerably reduce the dose to be administered to each patient during the treatment.

The best properties, in terms of SAR, of the cubic nanocrystals originate closely from the controlling of the shape, dimensions and polydispersion obtainable during their preparation. Thus far, the best qualitative results during the preparation of cubic nanocrystals were obtained by means of methods for the thermal decomposition of organometallic precursors in the presence of surfactants, as mentioned in patent application WO 2013/150496, Guardia et al. A PROCESS FOR PREPARING FERRITE NANOCRYSTALS by the same applicant, possibly also in the presence of structure directing agents, as described in the scientific publication "One pot synthesis of monodisperse water soluble iron oxide nanocrystals with high values of the specific absorption rate," by Guardia et al., J.Mater.Chem. B, 2014, 2,4426. Despite having optimum control of the magnetic and structural properties of the nanocubes, these methods make it possible to prepare a very small amount of material (a few tens of milligrams of iron nanocubes).

A further process for the preparation of magnetic nanoparticles is known from US 2005/191231 A1.

Solvothermal methods for preparing nanoparticles for magnetic hyperthermia have recently been proposed in the literature. In particular, Raja Das et al. in "Tunable High Aspect Ratio Iron Oxide Nanorods for Enhanced Hyerthermia," published in J.Phys.Chem.C 2016 120, 10086-10093, report the synthesis of nanorods of Fe₃O₄ from an organometallic precursor, a surfactant and an organic base in the presence of 1-octanol as the solvent, after 6 hours at 200°C in an autoclave. In a preliminary study mentioned in "Solvothermal Synthesis of Tunable Electroactive Magnetite Nanorods by Controlling the Side Reaction," J. Phys. Chem. C 2012 116, 5476-5481, Sun et al. observed the formation of FeO (wüstite) nanocubes after a reaction period of 80 minutes, due to the decomposition and successive oxidation of Fe(CO)₅ used as a precursor. The formation of FeO was, however, considered to be an intermediate process that develops in the magnetite phase as a result of thermal treatment (annealing) with exposure to a temperature of at least 200°C for longer periods of time (from 6 to 24 hours), as confirmed by the absence thereof at the end of the synthesis process. A revision study conducted by Rafienia et al., "Solvothermal Synthesis of Magnetic Spinel Ferrites," published in J. Med. Sign. Sens. 2018;8: 108-18, identified a few critical parameters for the solvothermal methods, such as the operating temperature (in the range of 100°C-240°C) and the need for long reaction times to obtain products having regular shapes. In particular, the authors cite a preparation of magnetite nanocubes having a narrow dimensional distribution and obtained in hydroalcoholic solvent after a reaction period of 24 hours at 230°C and in the presence of polyethylene glycol (PEG) and sodium dodecyl sulfate (SDS) as directing agents. However, none of the methods cited above makes it possible to obtain a good yield of magnetic nanoparticles having a high specific absorption rate and in a manner that may be implemented on a large scale. In the field of magnetic hyperthermia, there is therefore still a strong need to provide methods for preparing magnetic nanoparticles having optimum magnetic and colloidal properties for clinical application, which methods are easy to implement on a large scale.

The object of the present invention is to provide a synthesis method that overcomes the problems cited above.

In view of this object, the subject-matter of the invention consists of a method for preparing magnetic nanoparticles, which has the features defined in the following claims.

Additional advantages and the features of the method of the invention will become clear from the following description regarding both the general method features and specific embodiments.

### Brief description of the drawings

In the attached drawings:

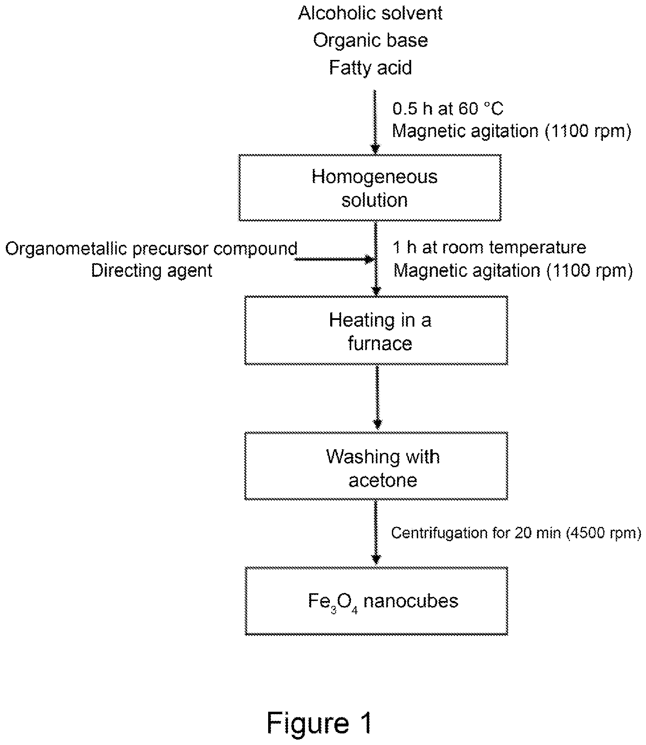

Fig. 1 is a block diagram of the synthesis method used in Example 1.
Fig. 2 shows the results of the TEM (a) and XRD (b) characterizations carried out on a sample of Fe₃O₄ nanocubes prepared according to Example 1.
Fig. 3 shows the results of the TEM characterizations carried out on samples prepared according to Examples 1 and 2.
Fig. 4 shows the results of the TEM characterizations and a diagram of the average dimensions obtained by DLS measurements (a, c) and XRD characterization (b, d) carried out on samples prepared according to Example 3 of mixed ferrite nanocubes of zinc or manganese, respectively.
Fig. 5 shows the results of the TEM and DLS analyses obtained according to Example 4. In particular, 4a shows the results of the sample synthesized according to the method of the prior art cited in Example 4. 5b shows the results for the sample according to Example 1. 5c shows the hydrodynamic dimensions weighted by intensity for each sample. 5d shows a table of the hydrodynamic dimensions measured.
Fig. 6 shows the results of the TEM, DLS and XRD analyses obtained according to Example 5. In particular, 5a shows the results for the sample synthesized according to the method of the prior art cited in Example 5. 6b shows the results for the sample according to Example 1. 6c shows the diffractograms recorded for the sample according to the method of the prior art (top) and the method according to Example 1 (bottom). 6d shows the hydrodynamic dimensions weighted by intensity for each sample in aqueous solution. 5e shows a table of the hydrodynamic dimensions measured.

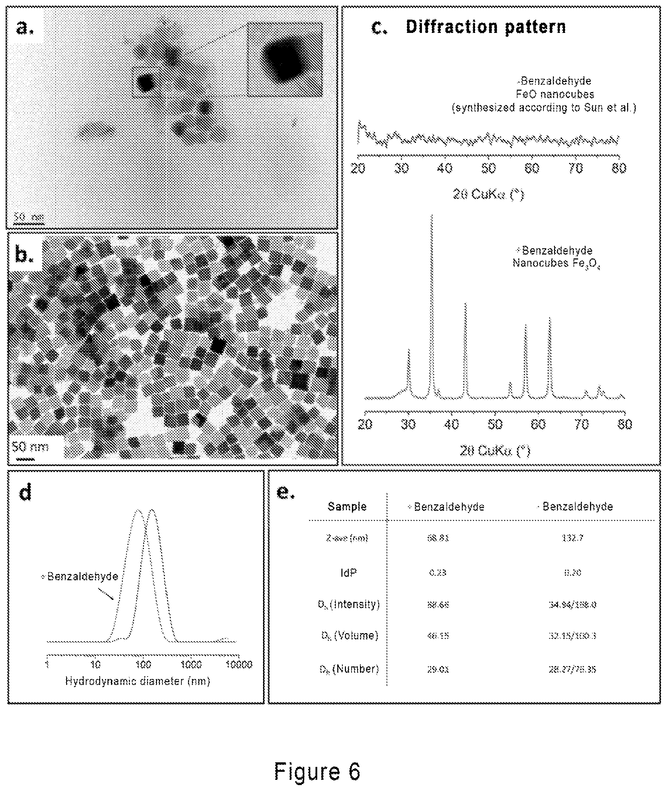

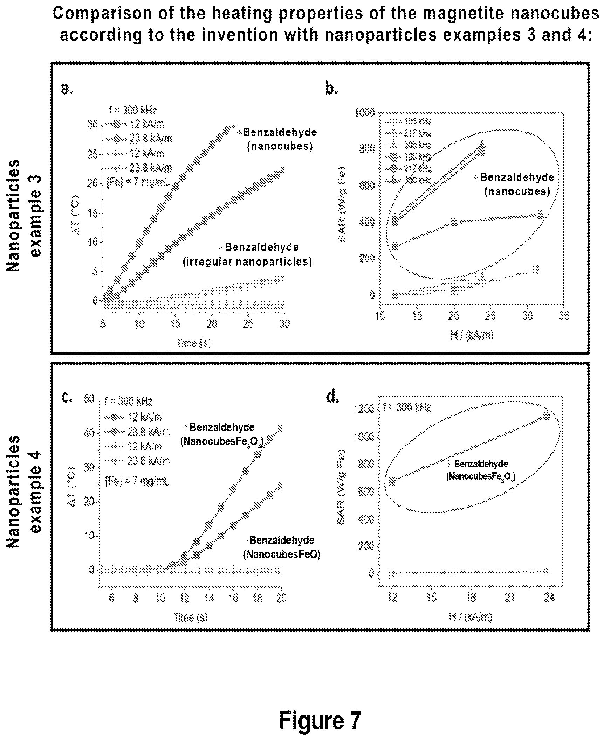

Fig. 7 shows the results of the tests performed on the magnetic properties of the cubic nanocrystals obtained according to the invention and of the comparative nanoparticles. In particular, Fig. 7a shows the temperature variation values as the time increases for the nanoparticles according to the invention and the magnetite nanoparticles, which have an irregular shape, obtained according to Example 3 and subjected to a magnetic field having a frequency of 300 kHz and a variable intensity. Fig. 6b shows the corresponding SAR values measured for different magnetic field intensities (12-23.8 kAm⁻¹) and different frequencies (100-300 kHz). Fig. 6c shows the temperature variation values as the time increases for the nanoparticles according to the invention and the FeO nanoparticles obtained according to Example 5 and subjected to the same magnetic field having a frequency of 300 kHz and intensity of 12-23 kAm⁻¹. Fig. 6d shows the corresponding SAR values calculated for different intensities of the magnetic field (12-23 kAm⁻¹) and a frequency of 300 kHz.

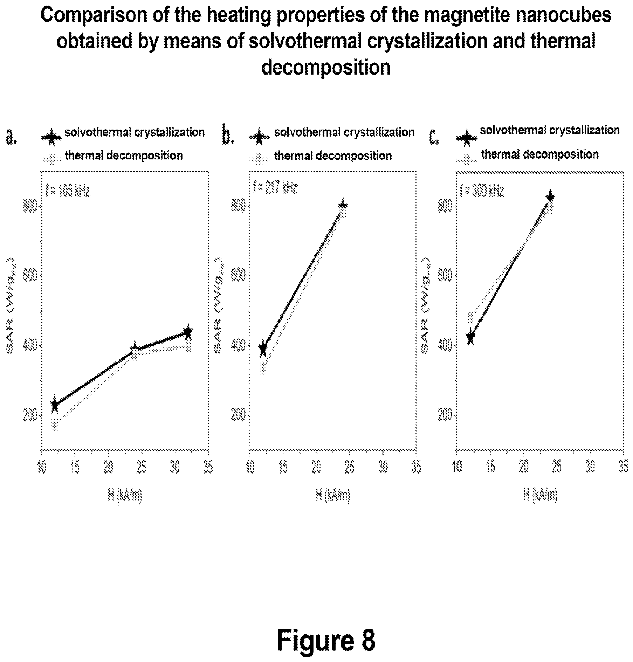

Fig. 8 shows the results of the tests performed on the magnetic properties of the cubic nanocrystals obtained according to the present invention and those obtained by means of thermal decomposition according to the method in the above-cited publication by Guardia et al. In particular, Fig. 8a to 8c show the SAR values as the magnetic field applied varies (in the range of 12-32 kAm⁻¹), which values are obtained at different frequencies (105 kHz, 217 kHz and 300 kHz, respectively) for samples of nanoparticles having comparable dimensions (21 ± 2 nm for the nanocubes according to the invention and 24 ± 2 for the reference nanocubes).
Fig. 9 shows the reactors used, the TEM analyses and the measurements of the dimensions of the corresponding products obtained by means of the experiments according to Example 7 in a table.
Fig. 10 shows the graphs showing the trend of the dimensions of the nanocrystals obtained according to Example 7 as some of the reaction parameters change. In particular, in 10a, the variable considered is the filling percentage of the reactor, while in 10b it is the reaction temperature.
Fig. 11 shows the results of the characterizations carried out for the samples subjected to ligand exchange according to Example 8. The DLS characterization results are shown in a and d, b shows TEM images, c shows the Z potential measurements and e, f and g show the SAR values.
Fig. 12 shows in a diagram of the method followed for the functionalization of the samples according to Example 9, and in b, c and d shows the results of the relative characterizations.

### Detailed description of the invention

For the purposes of the present description, the following terms are understood as having the following meanings.

"Directing agent" indicates a compound that is able to influence the growth of a nanoparticle to assume a predefined shape. Examples of common directing agents are polymers, surfactants, ionic salts and aromatic organic molecules.

"Precursor" indicates a chemical species containing at least one of the metal elements necessary for the nucleation/growth of the ferrite nanocrystals.

"Ligand" indicates a chemical species having surfactant properties that are able to form a compound with a metal.

"Ferrite" indicates a chemical compound consisting of a mixture of iron oxides and optionally of oxides of other metals selected from Fe, Mn, Co and Zn, having a high degree of magnetic permeability.

"Thermoresponsive polymer" indicates a polymer that exhibits a drastic change in physical properties as the temperature changes.

In a first aspect, the present invention relates to a method for producing ferrite nanocrystals, comprising the following steps:
i) providing a solution comprising a fatty acid, an aliphatic amine and an alcoholic solvent;
ii) adding at least one organometallic precursor compound comprising a metal selected from Fe, Mn, Co or Zn and an aromatic aldehyde as defined below to the solution in point
i) in order to obtain a reaction mixture;
iii) transferring the reaction mixture obtained in step ii) to a sealed reactor, thereby obtaining a filling percentage thereof of between 20 and 70 vol.%; and
iv) heating said sealed reactor to a temperature of between 160°C and 240°C for at least 3 hours.

In order to prepare the solution in step i), the components may be added under magnetic agitation and heating in order to facilitate the dissolution thereof and the attainment of a homogenous solution.

Said fatty acid is preferably a saturated or unsaturated fatty acid having an alkyl chain having between 10 and 18 carbon atoms. In a particularly preferred embodiment, said fatty acid is selected from oleic acid and decanoic acid.

Said aliphatic amine may be a primary, secondary or tertiary amine. Said aliphatic amine preferably has an alkyl chain having between 8 and 18 carbon atoms. Even more preferably, said aliphatic amine is hexadecylamine.

Said alcoholic solvent is selected from linear alcohols having an alkyl chain of between 2 and 8 carbon atoms and benzyl alcohol. In a particularly preferred embodiment, said alcohols are selected from 1-butanol and 1-octanol.

In step ii), the components may be added in the presence of magnetic agitation and at a temperature of between room temperature and the dissolution temperature used in step i).

In a particularly preferred embodiment, said organometallic precursor compound is selected from iron pentacarbonyl of formula Fe(CO)s, zinc acetylacetonate of formula Zn(AcAc)₂, cobalt(II) acetylacetonate of formula Co(AcAc)₂, manganese acetylacetonate of formula Mn(AcAc)₂, and mixtures thereof.

Said aromatic aldehyde is selected from benzaldehyde, 4-biphenyl carbaldehyde, 2-phenylpropionaldehyde, 1,4-benzenedicarboxaldehyde, 4-methylbenzaldehyde, vinylbenzaldehyde, isopropenylbenzenaldehyde, 4-isopropylbenzaldehyde, 4-(1-methylethyl)benzaldehyde. Such aldehydes are low molecular weight molecules able to facilitate the anisotropic growth of nanocrystals In a particularly preferred embodiment, the aromatic aldehyde is benzaldehyde.

In the embodiments in which said aromatic aldehyde is in the solid state at room temperature and pressure, it may be added to step i) to facilitate the dissolution thereof in the reaction mixture.

Optionally, the above-mentioned aromatic aldehyde may be used in a mixture with directing agents, such as alkylamines or trioctylphosphine (TOPO).

In step iii), said sealed reactor is preferably a Teflon-lined autoclave (for operating temperatures of up to 200°C), or p-polyphenylene (PPL) (for temperatures above 200°C). Alternatively, any reactors on the market may be used that may be sealed and pressurized, for example Parr^{®} reactors. The use of a sealed reactor makes it possible to obtain an autogenous pressure inside said reactor and this pressure ensures the high degree of crystallinity of the end product, despite the reduced operating temperature. Extensive studies have made it possible to determine the importance of parameters such as the reactor volume and the filling percentage thereof on the end properties of the ferrite nanocrystals, as shown in Example 6. In particular, a general trend was found in which, as the degree to which the reactor is filled increases, the reaction yield decreases and the average diameter of the nanocrystals obtained increases, while, as the volume of the reactor increases, the yield increases and the average diameter decreases. Furthermore, as the reaction temperature increases, an increase in the average dimensions of the nanocrystals obtained is observed. In any case, as each of these parameters varies, that is the volume of the reactor, the filling percentage or the temperature, the monodispersion and the cubic shape of the nanocrystals are not influenced.

Preferably, said filling percentage of the reactor is between 40 and 55 vol.%.

The process of heating the reactor in point iv) is preferably carried out by inserting said sealed reactor into a pre-heated furnace at the solvothermal reaction temperature.

The reaction temperature is preferably between 200 and 240°C.

The reactor is preferably held at the reaction temperature for between 3 and 6 hours.

In a preferred embodiment, the ferrite nanocrystals obtained according to the method of the present invention are transferred to water by means of a ligand-exchange step or a polymeric covering step.

As is known in the above-cited document WO 2013/150496, for example, in order to facilitate the transfer of nanocrystals to the required final use solvents, the nanocrystals may be functionalized with suitable ligands. Advantageously, the nanocrystals obtained according to the method described above readily lend themselves to being subjected to a variety of ligand-exchange methods or to methods of polymeric covering on the surface, obtaining transfer rates close to 100% and stable dispersions even in the long term, as illustrated in Example 8.

The ligand exchange may advantageously be carried out using tetramethylammonium hydroxide, polyethylene glycol and derivatives thereof. Examples of polyethylene glycol derivatives are gallol polyethylene glycol and α-nitrodopamine-ω-carboxypoly(ethylene glycol).

The polymeric covering may advantageously be carried out using an amphiphilic polyanhydride. Examples of amphiphilic polyanhydrides are poly(maleic anhydride-alt-1-octadecene), poly(maleic anhydride-alt-1-tetradecene), poly(maleic anhydride-polyisobutylene).

In another preferred embodiment, the ferrite nanocrystals obtained according to the method of the present invention are subjected to functionalization with a radical polymerization initiator for a monomer or comonomers susceptible to forming a thermoresponsive or pH-responsive polymer.

As described in patent application WO 2018/138677 by the same applicant, for example, this type of ferrite nanocrystal functionalization makes it possible to develop multi-functional materials that may permit the controlled release of a drug. When functionalizing with thermoresponsive polymers, the heat generated by the magnetic nanoparticles during magnetic hyperthermia treatment may be used to bring the temperature of the solution to above the lower critical solution temperature (also called the LCST), thereby causing the contraction of the polymeric chain and facilitating the release of a previously charged drug.

Therefore, proceeding from the nanocrystals obtained according to the method of the present invention, it is possible to introduce a radical initiator on the surface of the particles by means of a ligand-exchange method and to thereby obtain macroinitiator particles to be used in the subsequent photoinduced polymerization step of the comonomers of a thermoresponsive polymer. In particular, Example 9 demonstrates how cubic ferrite nanocrystals that are obtained according to the method of the present invention and covered with a thermoresponsive polymer having an LCST close to 42°C by means of copper-mediated photoinduced free-radical polymerization make it possible to obtain a material that combines excellent heating properties with thermoresponsive characteristics in a biological environment.

The method described thus far is used in the large-scale preparation of nanocrystals for use in magnetic hyperthermia, with numerous advantages. In particular, said method makes it possible to obtain high yields of magnetic nanoparticles, while simultaneously maintaining a high level of control of the dimensions, dispersion and colloidal properties of the end product. More specifically, the method described here has obvious advantages over the thermal decomposition synthesis methods, since it does not require operating in an atmosphere devoid of oxygen and under magnetic agitation, thereby substantially reducing the times and costs of the entire production process. Furthermore, the thermal profile of the method developed here is more direct and simple; it in fact consists of just one heating step to temperatures below those used in the known thermal decomposition methods and reduced reaction times. With respect to the solvothermal synthesis methods, the method described here makes it possible to obtain cubic nanoparticles having a greater degree of crystalline purity, reduced dimensions and lower polydispersion, consequently having better magnetic and colloidal properties.

### Example 1: synthesis of cubic magnetite nanocrystals

8 mL (equal to 51 mmol) of 1-octanol (anhydrous, ≥ 99% Sigma-Aldrich), 0.2 g (equal to 0.8 mmol) of hexadecylamine (HDA, 98%, Sigma-Aldrich) and 0.6 mL (equal to 1.9 mmol) of oleic acid (OA, ≥ 99% , GC, Sigma-Aldrich) were brought into a homogenous solution in a two-neck round-bottom flask (25 mL, with an opening for the insertion of a thermocouple) at 60°C using a heating jacket (the temperature is simply monitored, but a specific ramp is not applied) for 30 minutes with magnetic agitation (1100 rpm). No condensing units are required, no specific pressure and the dissolution was carried out under atmospheric pressure. After this, the solution was left to cool down to room temperature (RT) naturally. 2 mL (equal to 14.8 mmol) of iron pentacarbonyl (Sigma-Aldrich, > 99.99%, purity in terms of metal traces) were then added at room temperature with magnetic agitation (1100 rpm). After 30 minutes, 1 mL of benzaldehyde (Sigma-Aldrich, ReagentPlus^{®}, ≥ 99%) was added as the directing agent. After 30 minutes, the solution was transferred to a Teflon-lined 25-mL autoclave that is filled up to 46.4 vol.% and is sealed in a stainless-steel jacket. The reactor was introduced into a furnace pre-heated to 200°C and maintained for 6 hours, where the sample was subjected to the solvothermal crystallization process. No type of magnetic agitation was applied during the solvothermal reaction in order to prevent the possible aggregation of the nanoparticles formed, which may happen due to the magnetic characteristics of the product. The pressure inside the reactor may reach values of between 1.2 and 60 bar. For example, the pressure reached inside a Parr^{®} reactor (Series 4560, 100 mL) was measured reaching values of between 20 and 60 bar during the reaction. At the end of the solvothermal reaction in the furnace, the reactor was left to cool down to room temperature naturally. The contents of the autoclave were then transferred to two 45-mL Falcon^{™} tubes with the aid of chloroform up to a volume of 15 mL. The Falcon^{™} tubes were subjected to ultrasound for 2 minutes, 30 mL of acetone were added, were briefly agitated and were subjected to centrifugation (4500 rpm for 20 min). After this, the supernatant was discarded and the product deposited in the Falcon^{™} tube was dispersed in 10 mL of chloroform in each tube for the subsequent characterization processes.

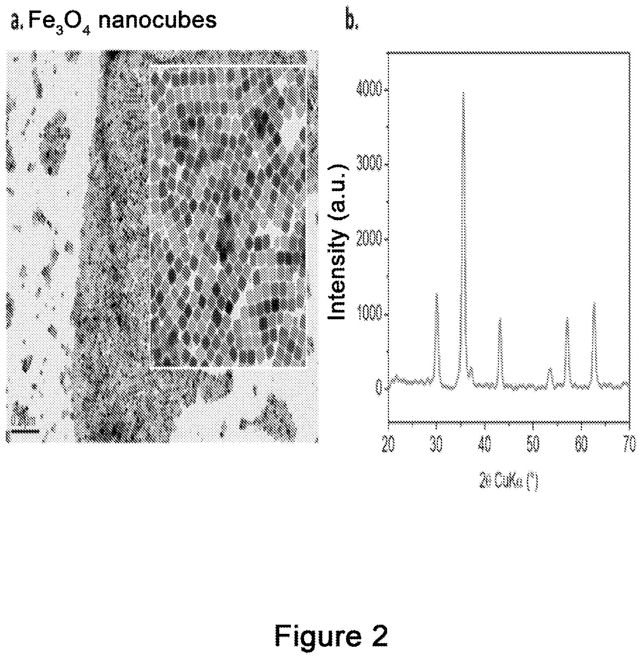

The nanocrystals obtained in this way were characterized by means of transmission electron microscopy (TEM). The results shown in Fig. 2a show the presence of cubic shapes and a dimensional distribution centered on 17 ± 2 nm was obtained. The sample was further characterized by means of X-ray diffractometry measurements recorded for angular values 2θ in the range of from 20 to 80° (Cu Kα) and the diffractogram obtained and shown in Fig. 2b confirms the presence of the spinel structure that is characteristic of the ferrites.

### Example 2: synthesis of cubic nanocrystals using different directing agents.

The procedure in Example 1 was reproduced in two separate experiments by substituting the directing agent with equimolar quantities of 2-phenylpropionaldehyde and 4-biphenyl carbaldehyde, respectively. In the first case, 1.31 mL of 2-phenylproprionaldehyde (equal to 9.8 mmol) (Sigma-Aldrich, 99%) were added as the directing agent, while in the case of 4-biphenyl carbaldehyde (Sigma-Aldrich, 98%), this compound being in the solid state in contrast to the preceding compounds, 1.82 g of 4-biphenyl carbaldehyde (equal to 9.8 mmol) were directly added to the initial reaction mixture, together with 1-octanol, hexadecylamine and oleic acid, and the mixture was heated to 60°C for 30 minutes.

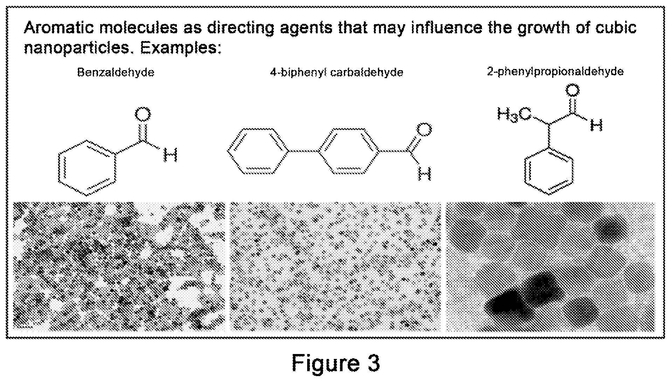

The results of the TEM characterization shown in Fig. 3 show the attainment of cubic nanoparticles irrespective of the directing agent used. A dimensional distribution centered on 17 ± 2 nm was also observed for benzaldehyde; 9 ± 1 nm for 4-biphenyl carbaldehyde and 19 ± 2 nm for 2-phenylprionaldehyde.

### Example 3: synthesis and characterization of cubic manganese, cobalt or zinc ferrite nanocrystals

Mn, Co or Zn ferrite nanocrystals were obtained by introducing a non-stoichiometric mixture of iron pentacarbonyl, Fe(CO)s and zinc acetylacetonate, Mn(AcAc)₂, Co(AcAc)₂ or Zn(AcAc)₂.

The experimental procedure carried out is the same as in Example 1 for the preparation of magnetite nanocubes, with the exception of a single modification. After the dissolution of oleic acid and hexadecylamine at 60°C, the metallic precursors were added at this temperature: initially, 0.5 mmol of manganese (II), cobalt (II) or zinc (II) acetylacetonate were dissolved (under magnetic agitation at 1100 rpm for 30 minutes) and then 14.3 mmol of iron pentacarbonyl. Benzaldehyde was then added and the solution was subjected to the solvothermal reaction as described in Example 1.

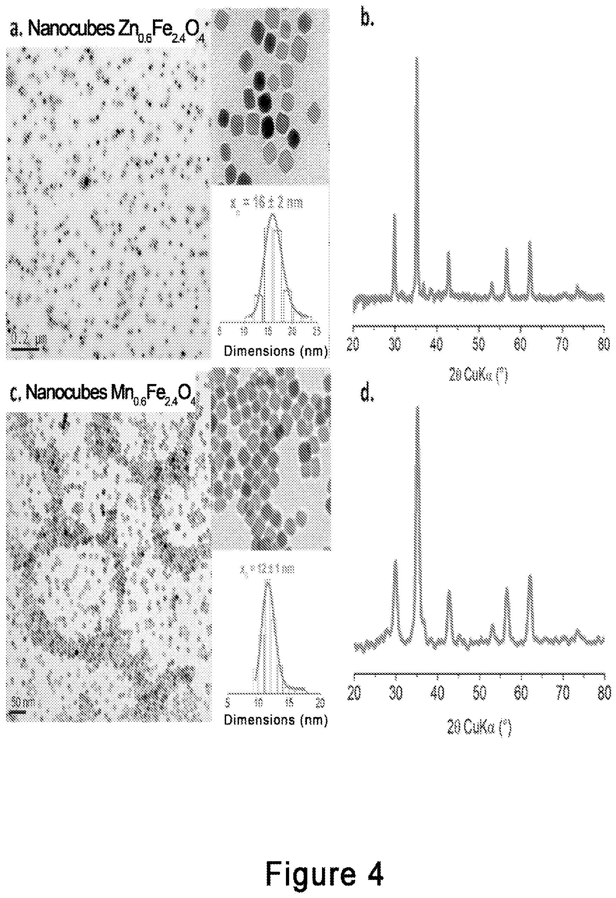

The nanocrystals obtained using Zn(AcAc)₂ obtained in this way were characterized by means of transmission electron microscopy (TEM) and by means of dynamic light scattering (DLS) measurements to determine the shape and dimensions thereof. The results obtained and shown in Fig. 4a show the presence of a cubic shape and a dimensional distribution centered on 16 ± 2 nm.

The sample was then characterized by means of X-ray diffractometry recorded for angular values 2θ in the range of 20-80° (Cu Kα) and the diffractogram obtained and shown in Fig. 4b confirms the presence of the spinel structure that is characteristic of the ferrites.

The elementary analysis was carried out by means of inductively coupled plasma atomic emission spectroscopy (ICP-AES) in order to identify the chemical composition of the sample, which turned out to be Zn_{0.6}Fe_{2.4}O₄.

In the case of manganese ferrite nanocubes obtained using Mn(AcAc)₂ as the precursor, the average diameter of the nanocubes is 12 ± 1 nm, as observed in Fig. 4c. The diffractogram obtained and shown in Fig. 4d confirms the presence of the spinel structure that is characteristic of the ferrites in all cases. The elementary analysis confirms a chemical composition corresponding to Mn_{0.6}Fe_{2.4}O₄.

### Example 4: comparative study of the colloidal properties.

By means of this study, the crucial role that the presence of the directing agent plays on the shape, polydispersion and colloidal stability of the nanoparticles obtained according to the method described here was established.

In this regard, the experimental procedure described in Example 1 was repeated without the addition of benzaldehyde as the directing agent, and the structural and colloidal properties were compared by means of TEM and DLS analyses of the nanoparticles obtained according to the two synthesis methods.

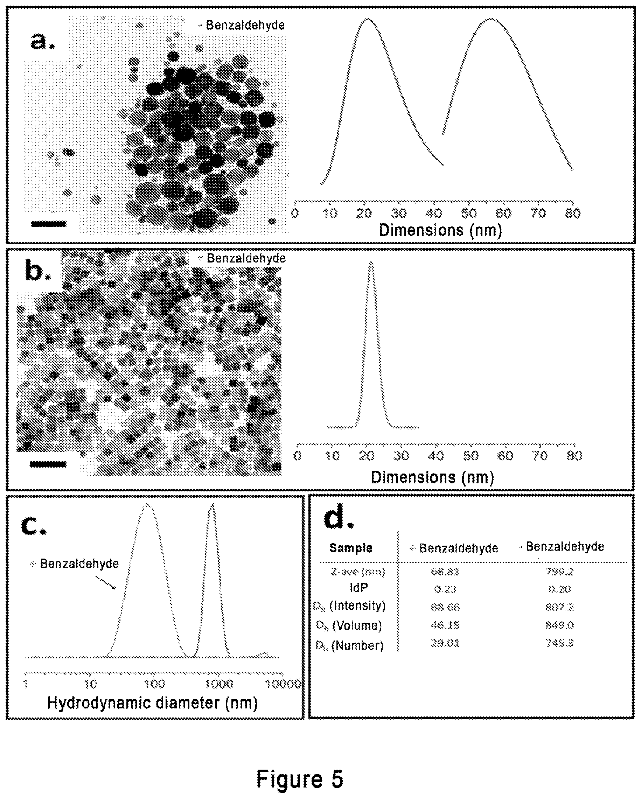

As may be seen in Fig. 5a, the results clearly showed that the synthesis process without the directing agent leads to a polydispersion system of spherical nanoparticles having irregular faces, in which two separate populations were identified: the first having dimensions of 23 ± 9 nm and the second having dimensions of 53 ± 14 nm. In contrast, in Fig. 5b, monodisperse cubic nanoparticles having dimensions of 21 ± 2 nm may be seen.

In order to compare the colloidal properties, the same ligand-exchange protocol using tetramethylammonium hydroxide (TMAOH) was applied to both samples. This is a short ligand (L) that may replace the organic surfactant on the surface of the cubic nanocrystal, providing a negative charge having a physiological pH that are able to improve the stability by means of charge repulsion. By means of experimentation, the synthesized samples were collected in a glass vial by means of centrifugation (4500 rpm for 20 minutes), dried by means of an air flow and an ethanol solution containing the ligand was added. The solutions were treated with ultrasound for 30 minutes and the solvent was then exchanged for water using an Amicon^{®} centrifugal filter (100-K MWCO). For the ligand-exchange process, 200 L/nm² were used. Only with the sample prepared using benzaldehyde during the solvothermal process did the stability of the nanocubes in an organic phase prove to be very good, and therefore the ligand exchange was direct and worked without any complications. The stability of both the samples after the ligand exchange was evaluated by hydrodynamic dimension measurements using the DLS technique. As shown in Fig. 5c and 5d, the nanocrystals prepared using benzaldehyde have a hydrodynamic dimension centered on 68 ± 15 nm, while the nanoparticles synthesized in the absence of benzaldehyde show dimensions centered on 799 ± 160 nm. Furthermore, the dimensions of the nanocrystals determined by means of DLS in the aqueous means is in good agreement with those measured in the TEM analysis, thereby indicating that, unlike the comparative sample, the nanoparticles prepared using benzaldehyde are permanently dispersed in water after the transfer. Lastly, the nanoparticle colloids obtained according to Example 3 show long-term stability despite the presence of a short ligand. It is believed that this property is attributable to the stabilization by means of charge repulsion, which was confirmed by the Z potential measurements obtaining the value of-30 ± 2 mV.

### Example 5: comparative example of solvothermal process

The products obtained by means of the solvothermal method of the present invention were compared with the nanocubes obtained by means of a solvothermal method by Sun et al. (cited above).

According to the procedure by Sun et al., 0.20 g (0.8 mmol) of hexadecylamine and 2.0 mL (6.33 mmol) of oleic acid were added to 8.0 mL of 1-octanol and the mixture was heated to approximately 50°C under magnetic agitation to form a homogenous solution. After this, the solution was cooled down to room temperature and 2.0 mL (14.8 mmol) of Fe(CO)s were added. The mixture continued to be agitated and was transferred to a 25-mL Teflon autoclave. The autoclave was introduced into a pre-heated furnace at 200°C for 80 minutes. After cooling to room temperature, the precipitate was separated by means of centrifugation and dispersed in chloroform. The two methods differ significantly in the ratio between [oleic acid] [hexadecylamine], which is 2.4 in the method of the invention and 7.9 in the reference method, in the duration of the solvothermal crystallization process, which is 6 hours in the invention and 80 minutes in the reference method, and above all in the presence of benzaldehyde. At the end of the synthesis process according to the reference method, a small amount of material was retrieved as the precipitate at the bottom of the reactor and had an ochre color in contrast to the black color of the precipitate obtained according to the invention. The nanoparticles obtained according to the reference method are predominantly nanocubes having average dimensions of 26 ± 2 nm in accordance with what stated by the authors and having the dimensions that are obtainable according to the present invention (21 ± 2). However, as may be seen in the TEM images shown in Fig. 6a and 6b, the morphology of the nanoparticles obtained according to the invention is better defined, whereby the face of the cubic shape is clearly visible. Furthermore, as may be seen from the diffractograms shown in Fig. 5c, the nanoparticles obtained according to the reference method are amorphous. In contrast, the nanoparticles obtained according to the invention have a diffractogram that may be clearly attributed to the inverted spinel structure of the magnetite. Lastly, the ligand-exchange protocol described in Example 3 using tetramethylammonium hydroxide was applied to both samples to transfer the products to an aqueous means. Fig. 4d shows the hydrodynamic dimensions with weighted intensity for each sample measured using the DLS technique. The nanoparticles obtained according to the method proposed here show hydrodynamic dimensions of 68 ± 16 nm compared with 132 ± 26 for the other sample. Furthermore, the "shoulder" in the hydrodynamic radius dimensions of around 30 nm is also worth noting, which shows the heterogeneity of the sample.

### Example 6: comparative study of the magnetic performance

The magnetic performance (SAR) and heating behavior of the cubic magnetite nanocrystals obtained according to Example 1 were studied and compared by applying an AC magnetic field and recording the temperature increase verified by the different colloidal nanoparticle systems. AC magnetic fields having different frequencies (105 kHz, 220 kHz and 300 kHz) and amplitudes of up to 32 kAm⁻¹ were applied. All measurements were carried out in water (C_{water} = 4185 JL⁻¹K⁻¹). The SAR values recorded were calculated by the average of four measurements on one sample having a volume of 0.3 mL and an iron concentration of 4-7 mg/mL in order to guarantee measurement conditions in which heat dissipation is close to adiabaticity. Only the first seconds were used to calculate the gradient of the curve and the SAR values.

The heating properties of the magnetite nanocubes according to the invention were compared with: i) the corresponding nanoparticles having an irregular shape obtained in the absence of a directing agent according to Example 4; ii) the FeO nanocubes prepared according to Example 5, and iii) nanocubes obtained by means of thermal decomposition according to the method described in the above-cited publication by Guardia et al..

As may be seen from the results shown in Fig. 7, the cubic nanocrystals prepared according to the method of the invention are able to cause a significant increase in the temperature within a few seconds, different from the nanoparticles having an irregular shape (obtained without benzaldehyde). This phenomenon is clearly reflected in the SAR values calculated, which, for the nanoparticles in Example 1, are above 200 W/g Fe: in fact, as the intensity and the frequency of the magnetic field applied increase, said values vary from 30 to 1100 W/g. In comparison, the value for the other sample is below 200 W/g Fe due to both the high degree of polydispersion in terms of the dimensions and shape and the very poor colloidal stability. For further comparison, the values stated in the work by Raja Das et al. for magnetite nanorods (produced by the solvothermal method), magnetite nanocubes and nanospheres (produced using a thermal decomposition method) having a significantly lower degree of heating efficiency and thereby obtaining 80-100 W/g as the best result (f = 310 kHz, H= 300 Oe, [Fe]= 3 mg/mL) are considered.

The same type of comparison, shown in Fig. 7 c and d, for the FeO nanocubes obtained according to the method described by Raja Das et al. and repeated in Example 5. As expected from the low degree of crystallinity of this product and from the antiferromagnetic structure of the wüstite, no rise in the temperature was observed and the SAR values are equal to zero for any variation in the magnetic field applied.

Furthermore, the heating properties of the magnetite nanocubes according to the present invention were compared, following the same techniques mentioned above, with the nanocubes obtained by thermal decomposition according to what described by Guardia et al. As may be observed from the results shown in Fig. 8, the SAR behavior of the two systems appears to be similar within the limits of experimental error.

At the end of the synthesis process, these products also appeared to be homogenously dispersed and easily transferable to other organic or aqueous means. In the case of the aqueous means, tetramethylammonium hydroxide (TMAOH) was used as the stabilizing agent following a ligand-exchange process. Following the same process, the mixed Zn and Mn ferrite nanocubes were also transferred to water and then used for the calorimetric measurements.

As may be seen from the data shown in Fig. 7 and 8, the SAR values measured for all the ferrites obtained according to the present invention appear to be promising for clinical application.

### Example 7: Control studies of the dimensions

In these experiments, the effects of i) the volume of the reactor and the filling percentage thereof and ii) the reaction temperature on the dimensions of the end product were examined separately.

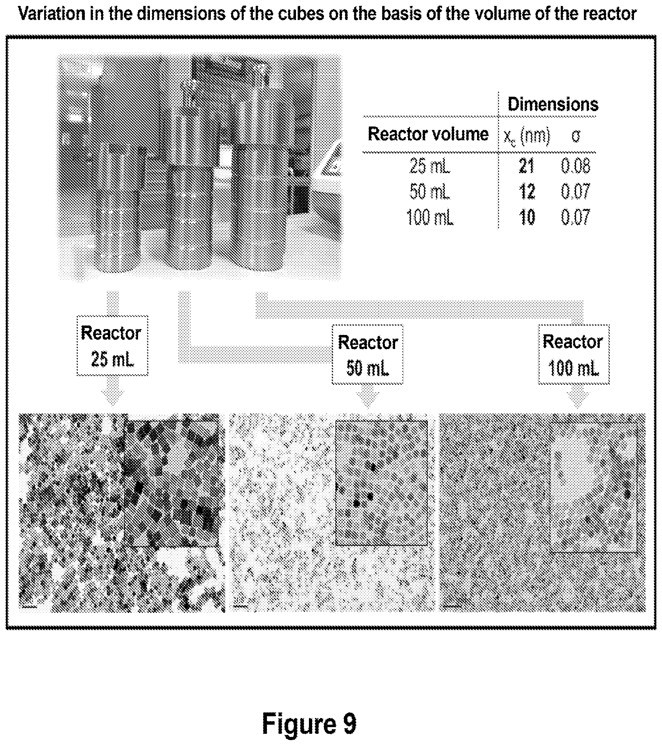

The study of the effect of the volume was carried out by repeating the experimental conditions in Example 1 in autoclaves having a volume of 50 and 100 mL by multiplying the quantity of reagent used in Example 1, in which a 25-mL reactor was used, by a factor of 2 and 4, respectively. As may be seen from the data shown in Fig. 9, neither the shape nor the monodispersion of the nanocubes are affected by the variation in the volume, while it may be seen that the dimensions decrease as the volume of the reactor increases.

The following table shows the mass of iron values obtained for each reactor at different temperatures.

| Volume of the autoclave (mL) | Temperature (°C) | Mass of iron (mg) |
|---|---|---|
| 25 | 200 | 70 |
| 50 | 200 | 190 |
| 100 | 200 | 420 |
| 25 | 240 | 280 |
| 50 | 240 | 830 |
| 100 | 240 | 1100 |

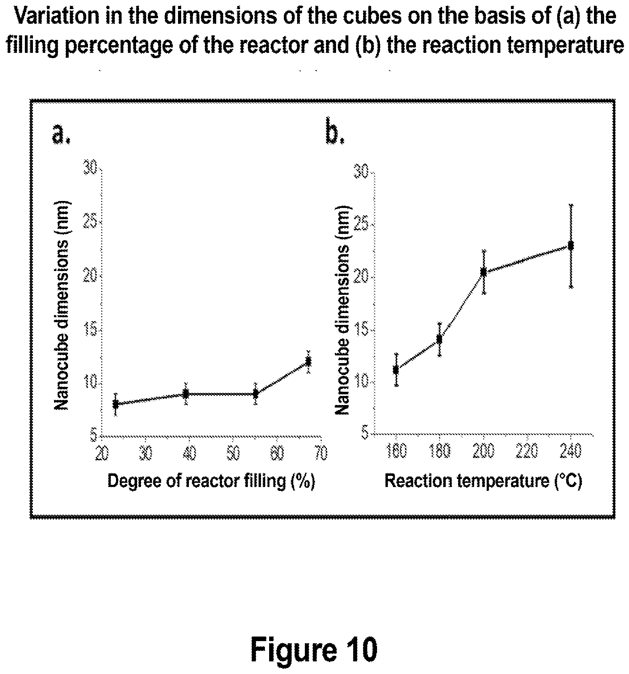

The effect of the filling percentage of the reactor was examined since, as it is known, this factor influences the internal pressure of the reactor, which in turn influences the dimensions of the nanoparticles and the final yield thereof (here expressed in terms of mass of Fe). For this purpose, tests were carried out in which the volume of the initial liquid phase varied from 10 to 32 mL in a 50-mL autoclave, thereby obtaining a corresponding variation in the filling percentage from 20 to 70%. As shown by the results in Fig. 10a, as the filling percentage of the autoclave increases, the cubic morphology is not influenced, while an increase in the average dimensions is observed.

From the point of view of the yield, it was observed that the method described here achieves a conversion into nanoparticles yield of up to 23% by mass of iron.

Lastly, it was found that, by setting the volume of the reactor to 25 mL and the filling percentage thereof to 46.4%, as the reaction temperature varies in the range of between 160 and 240°C, it is observed that an increase in the dimensions of the nanocubes in the range of between 11 and 23 nm was obtained, as shown in Fig. 10b.

### Example 8: ligand exchange

For this experiment, magnetite nanocrystals having average dimensions of 14 ± 1 nm, tetramethylammonium hydroxide (abbreviated as TMAOH or L1 here), gallol polyethylene glycol (abbreviated as GA-PEG or L2 here) α-nitrodopamine-ω-carboxypoly(ethylene glycol) (abbreviated as ND-PEG-COOH or L3 here) and poly(maleic anhydride-alt-1-octadecene) (abbreviated as POLY or P1 here) were used.

In order to carry out the exchange using L1, 15 mL of a chloroform solution containing magnetite nanocrystals where [Fe]=1 mg/mL were added to 8.2 mL of GA-PEG solution (0.1 M in chloroform containing 0.8 mL of triethylamine) and mechanically agitated overnight in an orbital agitator at room temperature. In this step, the ligand was found to have an excess of 300 L/nm². The mixture was then transferred to a separating funnel and the nanocrystals were transferred in a liquid phase by means of liquid-liquid extraction using water/toluene. The solution was concentrated up to 10 mL under reduced pressure conditions at 50°C, the excess ligand was removed by dialysis against 5 L of deionized water using a tube cellulose membrane with pore dimensions of 50 kDa. Lastly, the dispersion containing the nanocrystals was concentrated by means of centrifugation in a centrifugal filter (molecular threshold value of 100 kDa).

In order to test ND-PEG-COOH, 148 mg of polymeric ligand (0.098 mmol) were dissolved in 3.1 mL of chloroform to obtain a solution of 0.05 M. 0.67 mL of nanocrystals in chloroform (5 mg of Fe) were added. The final concentration was 1 mg Fe/mL and a ratio of 150 ligands/nm² was applied. 0.97 mL of triethylamine were then added (70 equivalents compared with the millimoles of ligands). The mixture was vigorously agitated overnight at room temperature to exchange the initial hydrophobic ligands with hydrophilic ND-PEG-COOH. 40 mL of toluene were then added and the nanocrystals were transferred in an aqueous phase by means of liquid-liquid extraction, as described above. After this, the sample was concentrated by means of centrifugation using an Amicon^{®} filter (50,000 g/mol threshold value, 1500 rpm). The sample was transferred to a dialyzer having a Spectrum Labs^{®} membrane (threshold value 100,000 g/mol, 1700 rpm) and the mixture was dialyzed for 2 days against water to purify it and further concentrated to a final volume of 2 mL.

Lastly, the nanocrystals were transferred to an aqueous means by means of polymeric covering with POLY. For this purpose, a polymer solution in chloroform was added to a dispersion of nanocrystals covered with oleic acid at a concentration of 0.01 µM in order to obtain a ratio of 500 L/nm². The solvent was then completely removed by means of rotary evaporation and the film of nanocrystals deposited on the walls was retrieved using a buffer solution of sodium borate (50 mM, pH 9). Lastly, the solution was concentrated in a tubular centrifuge equipped with a cellulose membrane and purified with a continuous sucrose gradient of 20-66%. All the samples were then subjected to TEM and DLS characterization, the Z potential was measured and the SAR values were calculated.

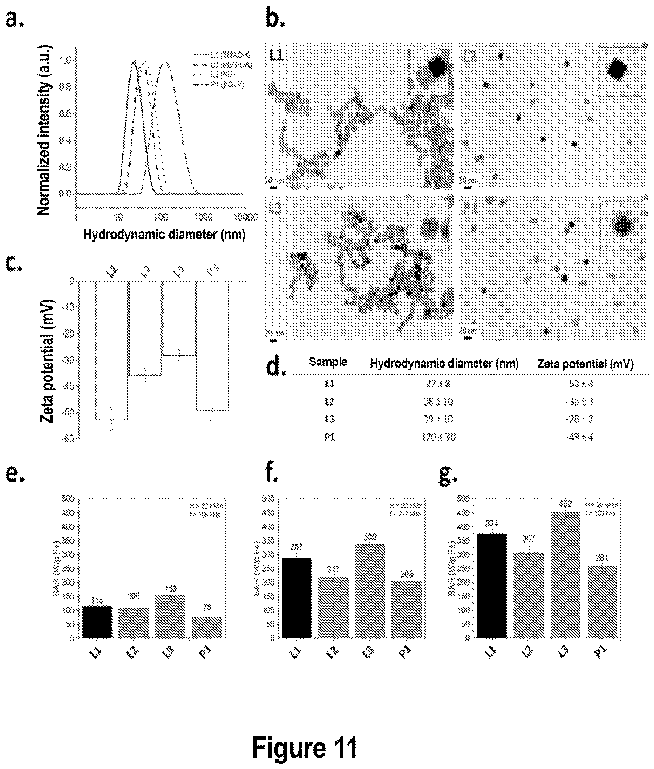

The hydrodynamic dimension measurements (weighed by intensity) shown in Fig. 11a are consistent with the sum of the dimensions of the nanocrystals and the average molecular weight of the ligands used (TMAOH < PEG-GA < ND-PEG-COOH) and are respectively centered on 27 ± 8 nm, 38 ± 10 nm and 39 ± 10 nm, while, for the polymeric shell, the value of 120 ± 30 nm was obtained. The TEM images shown in Fig. 11b show the stabilizing efficiency of the ligands, highlighting the absence of aggregates of nanocrystals, and, in the case of P1, the perfect shell covering of the nanocrystals.

The negative Z potential values measured for the different samples, shown in Fig. 11c, make it possible to deduce the presence of stabilization by means of charge repulsion, even in the long term. Furthermore, for POLY, this stabilization adds up to the steric stabilization specific to the presence of the polymer shell.

Lastly, the samples subjected to ligand exchange or polymeric covering were subjected to SAR measurements, the results of which are shown in Fig. 11 e-g. The measurements were carried out using a magnetic field having a fixed amplitude of 20 kA/m and an iron concentration in the samples of 5 mg/mL. The results obtained are similar to those obtained in Example 5 and with those mentioned by Guardia et al. in J. Mater. Chem. B, 2014, 2,4426 for nanocrystals having the same dimensions and PEG-GA as the ligand. Furthermore, all the samples made it possible to record the fast speed at which the temperature of 42°C was reached. Only in the case of the polymeric covering were the heating properties partially decreased, as is to be expected because of the greater hydrodynamic dimensions of this sample.

### Example 9: functionalization using thermoresponsive polymers

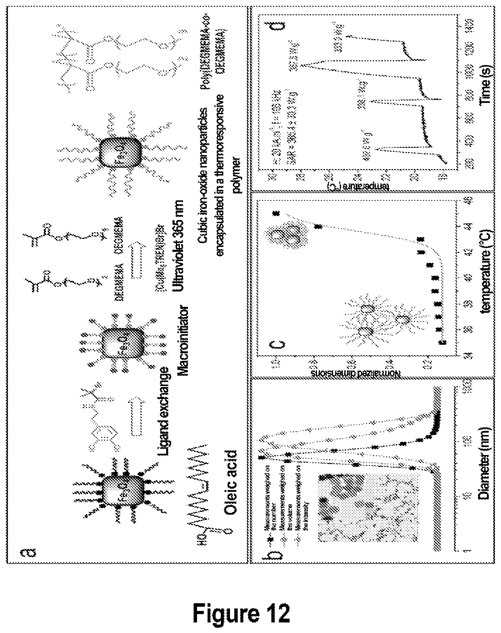

The functionalization procedure used in this example is shown schematically in Fig. 12a and the composition of the starting monomers (80% DEGMEMA and 20% OEGMEMA) was selected, in view of the clinical application, so as to have a polymer having an LCST close to 41°C.

Experimentally, magnetite nanocrystals having average dimensions of 20 ± 2 nm, diethylene glycol methyl ether methacrylate (DEGMEMA) (Aldrich, 95%) and oligoethylene glycol methyl ter methacrylate (OEGMEMMA) (M_{w} = 500 g mol⁻¹, Aldrich, 99%), both purified from the inhibitors by means of a basic alumina column, were used. The solvents were purchased from Sigma Aldrich with the highest degree of purity available and were used as received. Tris[2-(dimethylamino)ethyl]amine (ME₆TREN) was prepared according to the method described by Ciampolini and Nardi in "Five coordinated High Spin Complexes of Bivalent Cobalt, Nickel and copper with tris(2-dimethylaminoehyl)amine." Inorganic chemistry 5(1):41-44 (1966). 2-bromo-N-[2-(3,4-dihydroxy-phenyl)ethyl]-butyramide (DOPA-BiBA) was prepared according to the procedure described by Fan et al. in "Biomimetic anchor for surface-initiated polymerization from metal substrates," Journal of the American Chemical Society 127(45):15843-15847(2005).

The radical initiator was immobilized due to the high affinity of the catechol present in DOPA-BiBA towards the iron oxide surfaces, by means of a ligand-exchange procedure using an initial ratio of 500 ligand molecules per nm² of nanocrystal surface. In short, for cubic nanocrystals having a spike of 22.0 nm, the necessary amount of DOPA-BiBA was dissolved in 6.0 mL of methanol 4% (v/v) in chloroform inside a 20-mL flask. The required amount of cubic nanocrystals in chloroform was added to this solution and the suspension was subjected to treatment with ultrasound for 30 seconds. Triethylamine (TEA) was then added to the mixture in a stoichiometric ratio with DOPA-BiBA. The flask was covered with an aluminum sheet to prevent exposure to the light and left overnight, while being agitated. A surplus amount of hexane was then added to the mixture to induce the precipitation of the cubic nanocrystals. The precipitate was subjected to two dispersion cycles in tetrahydrofuran (THF)/precipitation in hexane to remove the unbound initiator. After this, the cubic nanocrystals were dispersed in THF to obtain an iron concentration of 4.0 gL⁻¹. For the actual polymerization step, 0.5 mL of the solution of cubic nanocrystals functionalized with DOPA-BiBA was diluted with 1.5 mL of dimethyl sulfoxide (DMSO) in a 40-mL flask. A mixture containing 2.5 mL of DMSO, 240 µL of OEGMEMMA and 448 µL of DEGMEMA was then slowly added to this solution, which was then treated with ultrasound for 30 seconds at room temperature and purged in nitrogen for 15 minutes. After this, 0.067 mg of a catalyst stock solution containing 0.5 mL of DMSO, 0.067 mg of CuBr₂ and 0,16 µL of ME₆TREN were injected into the flask. In order to start the polymerization process, the flask was held in a chilled environment at 5°C and exposed to a UV light source (four 9-W lamps, λₘₐₓ≈ 360 nm) while it continued to be agitated by an orbital agitator. After 2.5 hours of irradiation, the polymerization process was stopped by adding 10 mL of THF and exposing the entire thing to the air. The thermoresponsive cubic nanocrystals thus obtained were precipitated in diethyl ether and washed with two dispersion cycles in THF/precipitation in diethyl ether. The thermoresponsive cubic nanocrystals were then dissolved in water before the ultracentrifugation process using a sucrose gradient to remove the free polymer. The sucrose was removed by means of centrifugation on a filter and the stable thermoresponsive cubic nanocrystals were transferred to a pure saline solution for the characterization processes.

As may be observed from the DLS measurements shown in Fig. 12b, the thermoresponsive cubic nanocrystals obtained have a single-mode dimensional distribution and hydrodynamic dimensions of less than 100 nm, this data being indicative of the fact that the majority of the product obtained remains in solution in the individual state. This is further supported by the TEM images in the insert in Fig. 10b showing the nanocrystals functionalized with the thermoresponsive polymer as individual objects covered by a polymer shell that was determined to be 3-4 nm thinner than in the DLS measurement (20 nm). This discrepancy is explained by the dehydration process encountered by the polymeric chain once it has been deposited on the rack for the TEM analysis. Furthermore, by means of hydrodynamic dimension measurements, as the temperature increases, shown in Fig. 12c, an LCST of 44°C was determined.

Lastly, as shown in Fig. 12d, using exposure to an alternating magnetic field (H = 20 KAm⁻¹ and f = 105 kHz), it was possible to record a particularly high SAR value of 365 ± 30.3 W/g Fe.

All these data therefore confirm the possibility of functionalizing the nanocrystals obtained according to the present invention with a thermoresponsive polymer and obtaining multifunctional particles for biomedical applications able to combine magnetic hyperthermia and the controlled release of drugs.

## Claims

1. A method for preparing ferrite nanocrystals, comprising the following steps:
i) providing a solution comprising a fatty acid, an aliphatic amine and an alcoholic solvent;
ii) adding at least one organometallic precursor compound comprising Fe and optionally a second organometallic precursor compound comprising a metal selected from Mn, Co, Zn and an aromatic aldehyde selected from the group consisting of benzaldehyde, 4-biphenyl carbaldehyde, 2-phenylpropionaldehyde, 1,4-benzenedicarboxaldehyde, 4-methylbenzaldehyde, vinylbenzaldehyde, isopropenylbenzenaldehyde, 4-isopropylbenzaldehyde and 4-(1-methylethyl)benzaldehyde to the solution in point i) thereby obtaining a reaction mixture;
iii) transferring the reaction mixture obtained in step ii) to a sealed reactor, thereby obtaining a filling percentage thereof of between 20 and 70 vol.%; and
iv) heating said sealed reactor to a temperature of between 160°C and 240°C for at least 3 hours.

2. The method according to claim 1, wherein said aromatic aldehyde is benzaldehyde.

3. The method according to any one of the preceding claims, wherein said aliphatic amine in step i) is an alkyl amine.

4. The method according to any one of the preceding claims, wherein said fatty acid is a saturated or unsaturated fatty acid having an alkyl chain with a length of between 10 and 18 carbon atoms.

5. The method according to any one of the preceding claims, wherein said alcoholic solvent is selected from the linear alcohols having an alkyl chain of between 2 and 8 carbon atoms.

6. The method according to any one of the preceding claims, wherein said organometallic precursor compound is selected from iron pentacarbonyl of formula Fe(CO)s, zinc acetylacetonate of formula Zn(AcAc)₂, cobalt acetylacetonate of formula Co(AcAc)₂, manganese(II) acetylacetonate of formula Mn(AcAc)₂, and mixtures thereof.

7. The method according to any one of the preceding claims, wherein said filling percentage is between 40 and 55 vol.%.

8. The method according to any one of the preceding claims, wherein the temperature in step iv) is between 200 and 240°C.

9. The method according to any one of the preceding claims, wherein the nanocrystals obtained as a result of the synthesis process are transferred to water by means of a ligand-exchange step or a polymeric covering step.

10. The method according to claim 9, wherein the ligands used in said ligand-exchange step are selected from tetramethylammonium hydroxide, polyethylene glycol and derivatives thereof.

11. The method according to claim 9, wherein the polymer used in said polymeric covering step is an amphiphilic polyanhydride.

12. The method according to any one of claims 1 to 8, wherein the nanocrystals obtained as a result of the synthesis process are functionalized by a radical polymerization initiator for a monomer or comonomers that may form a thermoresponsive or pH-responsive polymer.

## Patentansprüche

1. Verfahren zum Herstellen von Ferrit-Nanokristallen, aufweisend die folgenden Schritte:
i) Bereitstellen einer eine Fettsäure, ein aliphatisches Amin und ein alkoholisches Lösungsmittel aufweisenden Lösung;
ii) Hinzufügen mindestens einer Fe aufweisenden organometallischen Vorläuferverbindung und optional einer zweiten organometallischen Vorläuferverbindung, aufweisend ein Metall, ausgewählt aus Mn, Co, Zn, und ein aromatisches Aldehyd, ausgewählt aus der Gruppe aufweisend Benzaldehyd, 4-Biphenylcarbaldehyd, 2-Phenylpropionaldehyd, 1,4-Benzoldicarboxaldehyd, 4-Methylbenzaldehyd, Vinylbenzaldehyd, Isopropenylbenzaldehyd, 4-Isopropylbenzaldehyd und 4-(1-Methylethyl)benzaldeyhd zu der Lösung von Punkt i), wodurch ein Reaktionsgemisch erhalten wird;
iii) Überführen des in Schritt ii) erhaltenen Reaktionsgemischs in einen versiegelten Reaktor, wodurch ein Befüllungsgrad desselben zwischen 20 und 70 Vol.-% erhalten wird; und
iv) Erhitzen des versiegelten Reaktors auf eine Temperatur zwischen 160°C und 240°C für mindestens 3 Stunden.

2. Verfahren nach Anspruch 1, wobei das aromatische Aldehyd Benzaldehyd ist.

3. Verfahren nach einem der vorangehenden Ansprüche, wobei das aliphatische Amin in Schritt i) ein Alkylamin ist.

4. Verfahren nach einem der vorangehenden Ansprüche, wobei die Fettsäure eine gesättigte oder ungesättigte Fettsäure mit einer Alkylkette einer Länge zwischen 10 und 18 Kohlenstoffatomen ist.

5. Verfahren nach einem der vorangehenden Ansprüche, wobei das alkoholische Lösungsmittel aus den linearen Alkoholen mit einer Alkylkette von zwischen 2 und 8 Kohlenstoffatomen ausgewählt ist.

6. Verfahren nach einem der vorangehenden Ansprüche, wobei die organometallische Vorläuferverbindung ausgewählt ist aus Eisenpentacarbonyl der Formel Fe(Co)₅, Zinkacetylacetonat der Formel Zn(AcAc)₂, Kobaltacetylacetonat der Formel Co(AcAc)₂, Mangan(II)acetylacetonat der Formel Mn(AcAc)₂ und Mischungen davon.

7. Verfahren nach einem der vorangehenden Ansprüche, wobei der Befüllungsgrad zwischen 40 und 55 Vol.-% liegt.

8. Verfahren nach einem der vorangehenden Ansprüche, wobei die Temperatur in Schritt iv) zwischen 200 und 240°C beträgt.

9. Verfahren nach einem der vorangehenden Ansprüche, wobei die infolge des Syntheseverfahrens erhaltenen Nanokristalle mittels eines Ligandenaustauschschritts oder eines Polymerabdeckungsschritts in Wasser überführt werden.

10. Verfahren nach Anspruch 9, wobei die in dem Ligandenaustauschschritt verwendeten Liganden ausgewählt sind aus Tetramethylammoniumhydroxid, Polyethylenglykol und Derivaten davon.

11. Verfahren nach Anspruch 9, wobei das in dem Polymerabdeckungsschritt verwendete Polymer ein amphiphiles Polyanhydrid ist.

12. Verfahren nach einem der Ansprüche 1 bis 8, wobei die infolge des Syntheseverfahrens erhaltenen Nanokristalle durch einen radikalen Polymerisationsinitiator für ein Monomer oder Comonomere, die ein wärmeempfindliches oder pH-empfindliches Polymer bilden können, funktionalisiert werden.

## Revendications

1. Procédé de préparation de nanocristaux de ferrite, comprenant les étapes suivantes :
i) la fourniture d'une solution comprenant un acide gras, une amine aliphatique et un solvant alcoolique ;
ii) l'ajout d'au moins un composé précurseur organométallique comprenant Fe et facultativement d'un second composé précurseur organométallique comprenant un métal sélectionné parmi Mn, Co, Zn et d'un aldéhyde aromatique sélectionné dans le groupe constitué du benzaldéhyde, du 4-biphénylcarbaldéhyde, du 2-phénylpropionaldéhyde, du 1,4-benzènedicarboxaldéhyde, du 4-méthylbenzaldéhyde, du vinyl-benzaldéhyde, de l'isopropènylbenzènaldéhyde, du 4-isopropyl-benzaldéhyde et du 4-(1-méthyléthyl)benzaldéhyde à la solution du point i) pour ainsi obtenir un mélange réactionnel ;
iii) le transfert du mélange réactionnel obtenu dans l'étape ii) vers un réacteur étanche, pour ainsi obtenir un pourcentage de remplissage de celui-ci entre 20 et 70 % en volume ; et
iv) le chauffage dudit réacteur étanche à une température entre 160 °C et 240 °C pendant au moins 3 heures.

2. Procédé selon la revendication 1, dans lequel ledit aldéhyde aromatique est le benzaldéhyde.

3. Procédé selon l'une quelconque des revendications précédentes, dans lequel ladite amine aliphatique dans l'étape i) est une alkylamine.

4. Procédé selon l'une quelconque des revendications précédentes, dans lequel ledit acide gras est un acide gras saturé ou insaturé ayant une chaîne alkyle d'une longueur entre 10 et 18 atomes de carbone.

5. Procédé selon l'une quelconque des revendications précédentes, dans lequel ledit solvant alcoolique est sélectionné parmi les alcools linéaires ayant une chaîne alkyle entre 2 et 8 atomes de carbone.

6. Procédé selon l'une quelconque des revendications précédentes, dans lequel ledit composé précurseur organométallique est sélectionné parmi le pentacarbonyle de fer de formule Fe(CO)₅, l'acétylacétonate de zinc de formule Zn(AcAc)₂, l'acétylacétonate de cobalt de formule Co(AcAc)₂, l'acétylacétonate de manganèse (II) de formule Mn(AcAc)₂, et des mélanges de ceux-ci.

7. Procédé selon l'une quelconque des revendications précédentes, dans lequel ledit pourcentage de remplissage est entre 40 et 55 % en volume.

8. Procédé selon l'une quelconque des revendications précédentes, dans lequel la température dans l'étape iv) est entre 200 et 240 °C.

9. Procédé selon l'une quelconque des revendications précédentes, dans lequel les nanocristaux obtenus à la suite du procédé de synthèse sont transférés dans de l'eau au moyen d'une étape d'échange de ligand ou d'une étape de recouvrement polymère.

10. Procédé selon la revendication 9, dans lequel les ligands utilisés dans ladite étape d'échange de ligand sont sélectionnés parmi l'hydroxyde de tétraméthylammonium, un polyéthylèneglycol et des dérivés de ceux-ci.

11. Procédé selon la revendication 9, dans lequel le polymère utilisé dans ladite étape de recouvrement polymère est un polyanhydride amphiphile.

12. Procédé selon l'une quelconque des revendications 1 à 8, dans lequel les nanocristaux obtenus à la suite du procédé de synthèse sont fonctionnalisés par un initiateur de polymérisation radicalaire pour un monomère ou des comonomères qui peuvent former un polymère thermosensible ou sensible au pH.
